# DEMANDE DE BREVET EUROPEEN

(11) **EP 3 398 924 A1**
(43) Date de publication de la demande: **07.11.2018**
(21) Numéro de dépôt: 18174658.7
(22) Date de dépôt: 27.03.2009
(51) Int. Cl.: C07C 17/25, C07C 21/18, C07C 17/354, C07C 19/08

(54) **PROCEDE POUR LA PREPARATION DU 2, 3, 3, 3-TETRAFLUORO-1-PROPENE**

(30) Priorité: 28.03.2008 FR 0801727
(62) Demande divisionnaire de: 09725078.1
(71) Demandeur: ARKEMA FRANCE, 92700 Colombes (FR)
(72) Inventeur: DEVIC, Michel, 69110 Sainte Foy Les Lyon (FR); GUILLET, Dominique, 69390 Vernaison (FR); GUIRAUD, Emmanuel, 69230 Saint-Genis-Laval (FR); WENDLINGER, Laurent, 69510 Soucieu en Jarrest (FR)
(74) Mandataire: Hirsch & Associés

(57) **Abrégé**

L'invention a pour objet un procédé continu en phase gaz de préparation de 2,3,3,3-tétrafluoro-1-propène comprenant les étapes suivantes: (i) hydrogénation de hexafluoropropylène en 1,1,1,2,3,3-hexafluoropropane; (ii) déhydrofluoration du 1,1,1,2,3,3-hexafluoropropane obtenu à l'étape précédente en 1,2,3,3,3-pentafluoropropène-1; (iii) hydrogénation du 1,2,3,3,3-pentafluoropropène-1 obtenu à l'étape précédente en 1,1,1,2,3-pentafluoropropane; et (iv) déhydrofluoration du 1,1,1,2,3-pentafluoropropane obtenu à l'étape précédente en 2,3,3,3-tétrafluoro-1-propène.

## Description

### DOMAINE DE L'INVENTION

L'invention a pour objet un procédé de préparation de composés fluorés, à savoir le composé fluoré 1234yf.

### ARRIERE-PLAN TECHNOLOGIQUE

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

On connaît plusieurs procédés de fabrication du 1234yf.

WO 2008/002499 décrit un procédé de production d'un mélange de 2,3,3,3-tétrafluoro-1-propène (HFO 1234yf) et de 1,3,3,3-tétrafluoro-1-propène (HFO 1234ze) par pyrolyse de 1,1,1,2,3-pentafluoropropane (HFC 245eb).

WO 2008/002500 décrit un procédé de production d'un mélange de 2,3,3,3-tétrafluoro-1-propène (HFO 1234yf) et de 1,3,3,3-tétrafluoro-1-propène (HFO 1234ze) par conversion catalytique de 1,1,1,2,3-pentafluoropropane (HFC 245eb) sur un catalyseur de déhydrofluoration.

Ces deux demandes précitées visent donc la production d'un mélange contenant une partie substantielle de produit 1234ze.

WO 2007/056194 décrit la préparation de 1234yf par déhydrofluoration de 245eb, notamment sur catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogenation of perfluoro-olefins", 1960, décrit de façon distincte diverses réactions chimiques sur des composes fluorés. Ce document décrit l'hydrogénation sensiblement quantitative de HFP sur un catalyseur à base de palladium supporté sur platine, la température passant de 20°C à 50°C, puis étant maintenue à cette valeur. Ce document décrit la déhydrofluoration du 1,1,1,2,3,3-hexafluoropropane (236ea) par passage au travers d'une suspension de KOH, pour produire du 1,2,3,3,3-pentafluoropropène-1 (1225ye). Ce document décrit l'hydrogénation du 1,2,3,3,3-pentafluoropropène-1 (1225ye) en 1,1,1,2,3-pentafluoropropane (245eb) sur un catalyseur palladium supporté sur alumine. Au cours de cette hydrogénation il se produit aussi une réaction d'hydrogénolyse, une quantité significative de 1,1,1,2-tetrafluoropropane étant produite. Ce document décrit la déhydrofluoration de 1,1,1,2,3-pentafluoropropane (245eb) en 2,3,3,3-tétrafluoro-1-propène (HFO 1234yf) par passage dans une suspension de KOH. Ces réactions sont décrites indépendamment les unes des autres, mêmes s'il est indiqué qu'il est possible de les combiner pour synthétiser une gamme de dérivés d'éthylène, propylène et isobutylène contenant des quantités variables de fluor.

Le document US-P-5396000 décrit la préparation de 1,1,1,2,3-pentafluoropropane par déhydrofluoration catalytique de 1,1,1,2,3,3-hexafluoropropane (236ea) en 1,2,3,3,3-pentafluoropropène-1 (1225ye), suivi d'une hydrogénation pour produire le composé recherché. La déshydrohalogénation du 236ea en 1225ye est effectuée en phase gazeuse, le produit de la réaction étant, dans un exemple, envoyé directement au réacteur suivant dans lequel a lieu l'hydrogénation du composé 1225ye en composé 245eb. Il est aussi indiqué dans ce document que le composé 236ea peut être obtenu par hydrogénation de hexafluoropropylène (HFP), en faisant référence au document Knunyants et al précité.

Le document US-P-5679875 décrit la préparation de 1,1,1,2,3-pentafluoropropane par déhydrofluoration catalytique de 1,1,1,2,3,3-hexafluoropropane (236ea) en 1,2,3,3,3-pentafluoropropène-1 (1225ye), suivi d'une hydrogénation pour produire le composé recherché. Les réactions sont effectuées en phase gaz. Il est aussi indiqué dans ce document que le composé 236ea peut être obtenu par hydrogénation de hexafluoropropylène (HFP), en faisant référence, parmi d'autres, au document Knunyants et al précité.

Il existe un besoin d'un procédé de préparation de 1234yf à partir d'un produit de départ qui soit aisément accessible, et qui conduise au produit recherché avec une sélectivité élevée, et avantageusement un rendement élevé.

### RESUME DE L'INVENTION

L'invention fournit donc un procédé continu en phase gaz de préparation de 2,3,3,3-tétrafluoro-1-propène comprenant les étapes suivantes:
(i) hydrogénation de hexafluoropropylène en 1,1,1,2,3,3-hexafluoropropane;
(ii) déhydrofluoration du 1,1,1,2,3,3-hexafluoropropane obtenu à l'étape précédente en 1,2,3,3,3-pentafluoropropène-1;
(iii) hydrogénation du 1,2,3,3,3-pentafluoropropène-1 obtenu à l'étape précédente en 1,1,1,2,3-pentafluoropropane;
(iv) déhydrofluoration du 1,1,1,2,3-pentafluoropropane obtenu à l'étape précédente en 2,3,3,3-tétrafluoro-1-propène.

Selon des modes de réalisation:
- l'hydrogène est introduit lors de l'étape (i) et/ou l'étape (iii) selon un ratio molaire surstoechiométrique.
- l'étape (ii) est mise en oeuvre en présence d'hydrogène, de préférence avec un ratio molaire H₂/produit à réagir compris entre 0,3 et 30, notamment entre 0,5 et 20, avantageusement entre 1 et 10, et l'étape (iv) est mise en oeuvre en l'absence d'hydrogène.
- l'étape (ii) est mise en oeuvre en présence d'hydrogène, de préférence avec un ratio molaire H₂/produit à réagir compris entre 0,3 et 30, notamment entre 0,5 et 20, avantageusement entre 1 et 10, et l'étape (iv) est mise en oeuvre en présence d'hydrogène, de préférence avec un ratio molaire H₂/produit à réagir compris entre 0,3 et 30, notamment entre 0,5 et 20, avantageusement entre 1 et 10.
- la quantité totale d'hydrogène est introduite lors de l'étape (i).
- le ratio molaire H₂/hexafluoropropylène est compris entre 2,3 et 30, avantageusement entre 3 et 20.
- le ratio molaire H₂/hexafluoropropylène est d'environ 2.
- le 1,1,1,2,3,3-hexafluoropropane n'ayant pas réagi au cours de l'étape (ii) est séparé après l'étape (ii) ou après l'étape (iii), mais avant l'étape (iv), et est éventuellement recyclé à l'étape (ii) et/ou à l'étape (i) .
- le 1,1,1,2,3,3-hexafluoropropane n'ayant pas réagi au cours de l'étape (ii) n'est pas séparé avant les étapes (iii) et (iv), du 1,2,3,3,3-pentafluoropropène-1 supplémentaire est obtenu au cours de l'étape (iv) à partir du 1,1,1,2,3,3-hexafluoropropane n'ayant pas réagi et ce 1,2,3,3,3-pentafluoropropène-1 supplémentaire est ensuite séparé et éventuellement recyclé à l'étape (iii) et/ou éventuellement l'étape (ii).
- les étapes d'hydrogénation (i) et (iii) sont mises en oeuvre dans le même réacteur, de préférence avec le même catalyseur, une étape de séparation étant éventuellement présente.
- les étapes de déhydrofluoration (ii) et (iv) sont mises en oeuvre dans le même réacteur, de préférence avec le même catalyseur, et dans lequel le procédé comprend en outre une étape de séparation séparant les produits issus dudit réacteur, notamment en une fraction contenant le 2,3,3,3-tétrafluoro-1-propène.
- le flux de l'étape (ii) contenant le 1,2,3,3,3-pentafluoropropène-1 est envoyé directement sans séparation vers l'étape (iii) au cours de laquelle se produit d'hydrogénation.
- les étapes (i), (ii) et (iii) sont mises en oeuvre dans le même réacteur, sur des lits de catalyseurs différents.
- les étapes (i), (ii) et (iii) sont mises en oeuvre dans trois réacteurs immédiatement en série sans séparation intermédiaire.
- à l'issue de cette étape (iii) on procède à une séparation, on récupère un flux de 1,1,1,2,3,3-hexafluoropropane et éventuellement de HF qui est recyclé à l'entrée du procédé et on récupère un flux de 1,1,1,2,3-pentafluoropropane et éventuellement d'hydrogène qui sont envoyés vers l'étape (iv).

### DESCRIPTION DETAILLEE DE MODES DE REALISATION

L'invention utilise quatre réactions en série, mises en oeuvre de façon continue, en phase gazeuse, les produits de réaction étant envoyés dans l'étape suivante, éventuellement après avoir subi un traitement par exemple de séparation, si besoin est.

Dans le procédé, les étapes réactionnelles sont mises en oeuvre en continu sur des flux en phase gaz. On obtient ainsi un procédé économique de préparation du composé 1234yf, le produit de départ, HFP, étant disponible facilement sur le marché, à un coût faible.

Les étapes d'hydrogénation sont mises en oeuvre de façon classique pour l'homme du métier. L'homme du métier pourra choisir les conditions opératoires pour que les réactions soient sensiblement quantitatives.

Les catalyseurs susceptibles d'être utilisés dans ces réactions sont ceux connus à cette fin. On peut notamment citer les catalyseurs à base d'un métal du groupe VIII ou rhénium. Ce catalyseur peut être supporté, par exemple sur carbone, alumine, fluorure d'aluminium, etc. ou peut ne pas être supporté, comme le nickel de Raney. Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium. Ces catalyseurs d'hydrogénation sont connus.

Le catalyseur peut être présent sous toute forme appropriée, par exemple sous forme de lit fixe ou fluidisé, de préférence en lit fixe. La direction de flux peut être de haut en bas ou de bas en haut. Le lit de catalyseur peut aussi comprendre une distribution particulière du catalyseur afin de gérer les flux de chaleurs générés par la réaction exothermique. Ainsi, il est possible de prévoir des gradients de densité de charge, de porosité, etc. du catalyseur afin de réguler l'exothermicité de la réaction. Par exemple, on peut prévoir que la première partie du lit comprend moins de catalyseur tandis que la seconde partie en comprend plus.

On peut aussi prévoir des étapes de régénération du catalyseur, de façon connue.

On peut aussi prévoir d'utiliser un gaz de dilution comme l'azote.

Les étapes d'hydrogénation sont exothermiques. La température de réaction peut être contrôlée à l'aide de moyens disposés à cet effet dans le réacteur, si besoin est. La température peut varier de quelques dizaines de degrés au cours de la réaction, la réaction (i) étant plus exothermique que la réaction (iii). Par exemple, la température d'entrée peut varier de 20°C à 150°C, et le gain en température peut varier de 5°C à 100°C.

Le temps de contact (rapport entre le volume de catalyseur et le flux total de la charge) est en général compris entre 0,1 et 100 secondes, de préférence entre 1 et 50 secondes et avantageusement entre 2 et 10 secondes.

La quantité d'hydrogène injectée peut varier dans de grandes mesures. Le ratio H₂/charge peut varier dans de grandes mesures, notamment entre 1 (la quantité stoechiométrique) et 30, notamment entre 1,5 et 20, avantageusement entre 3 et 10. Un ratio élevé conduira à une dilution, et donc une meilleure gestion de l'exothermicité de la réaction.

Les réactions de déhydrofluoration sont aussi mises en oeuvre de façon classique par l'homme du métier.

La réaction de déhydrofluoration peut être mise en oeuvre par passage dans une solution basique, notamment de KOH.

La réaction de déhydrofluoration est de préférence mise en oeuvre avec un catalyseur de déhydrofluoration. Ce catalyseur est par exemple un catalyseur à base d'un métal notamment d'un métal de transition ou un dérivé oxyde ou halogénure ou oxyhalogénure d'un tel métal. Des catalyseurs sont par exemple FeCl₃, oxyfluorure de chrome, Ni (incluant les treillis de Ni mesh), NiCl₂, CrF₃, et leurs mélanges. D'autres catalyseurs possibles sont les catalyseurs supportés sur carbone, les catalyseurs à base d'antimoine, les catalyseurs à base d'aluminium (tel que AlF₃ et Al₂O₃ et oxyfluorure d'aluminium et alumine fluorée), de palladium, de platine, de rhodium et de ruthénium. On pourra se référer à la liste donnée dans le document US-P-5396000, colonne 1, ligne 50 à colonne 2, ligne 2 ou à la liste donnée dans WO 2007/056194, page 16, lignes 13-23.

Selon une variante, on utilise un catalyseur mixte.

Ce catalyseur, contient à la fois du chrome et du nickel. Le ratio molaire Cr:Ni, par rapport à l'élément métallique, est généralement compris entre 0,5 et 5, par exemple entre 0,7 et 2, notamment voisin de 1. Le catalyseur peut contenir en poids de 0,5 à 20% de chrome et de 0,5 à 20% de nickel et, de préférence, entre 2 et 10% de chacun des métaux.

Le métal peut être présent sous forme métallique ou sous forme de dérivés, notamment oxyde, halogénure ou oxyhalogénure, ces dérivés, notamment halogénure et oxyhalogénure, étant obtenus par activation du métal catalytique. Bien que l'activation du métal ne soit pas nécessaire, elle est préférée.

Le support est à base d'aluminium. On peut citer plusieurs supports possibles comme l'alumine, l'alumine activée ou les dérivés d'aluminium. Ces dérivés d'aluminium sont notamment des halogénures ou oxyhalogénures d'aluminium, décrits par exemple dans US-P-4902838, ou obtenus par le procédé d'activation décrit ci-dessous.

Le catalyseur peut comprendre le chrome et du nickel sous une forme non-activée ou sous forme activée, sur un support qui a subi aussi l'activation du métal ou non.

Le catalyseur peut être préparé à partir d'alumine (en général une alumine dite activée; cette alumine activée est une alumine à porosité élevée, et est distincte de l'alumine ayant subi le traitement d'activation du métal). Dans une première étape l'alumine est transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200°C et 450°C, de préférence entre 250°C et 400°C). Le support est ensuite imprégné à l'aide de solutions aqueuses de sels de chrome et de nickel ou à l'aide de solutions aqueuses d'acide chromique, de sel de nickel et de méthanol (servant de réducteur au chrome). Comme sels de chrome et de nickel, on peut employer des chlorures, ou d'autres sels tels que, par exemple, les oxalates, formiates, acétates, nitrates et sulfates ou le bichromate de nickel, pour autant que ces sels soient solubles dans la quantité d'eau susceptible d'être absorbée par le support. Le catalyseur peut aussi être préparé par imprégnation directe de l'alumine (qui en général est activée) à l'aide des solutions des composés de chrome et de nickel ci-dessus mentionnées. Dans ce cas, la transformation d'au moins une partie (par exemple 70% ou plus) de l'alumine en fluorure d'aluminium ou oxyfluorure d'aluminium s'effectue lors de l'étape d'activation du métal du catalyseur.

Les alumines activées susceptibles d'être utilisées pour la préparation du catalyseur sont des produits bien connus, disponibles dans le commerce. Elles sont généralement préparées par calcination d'hydrates d'alumine (hydroxydes d'aluminium) à une température comprise entre 300°C et 800°C. Les alumines (activées ou non) peuvent contenir des teneurs importantes (jusqu'à 1000 ppm) de sodium sans que cela nuise aux performances catalytiques.

De préférence, mais sans que cela soit nécessaire, le catalyseur est conditionnée ou activé, c'est-à-dire transformé en constituants actifs et stables (aux conditions réactionnelles) par une opération préalable dite d'activation. Ce traitement peut être réalisé soit "in situ" (dans le réacteur de déhydrofluoration) ou bien dans un appareillage adéquat conçu pour résister aux conditions d'activation.

Cette étape d'activation comprend en général les étapes suivantes:
- Une étape de séchage. Cette étape de séchage est effectuée à haute température (250°C à 450°C, de préférence 300°C à 350°C) en général sous courant d'azote ou d'air. Cette étape peut être éventuellement précédée dans un premier temps d'une première étape de séchage à basse température (100°C à 150°C, de préférence 110°C à 120°C) en présence d'air ou d'azote. La durée de l'étape de séchage peut être comprise entre 10 et 50 heures.
- Une étape de fluoration. Cette étape de fluoration est mise en oeuvre à basse température (180°C à 350°C) au moyen d'un mélange d'acide fluorhydrique et d'azote, en contrôlant la teneur en HF de façon que la température ne dépasse pas 350°C. La durée de l'étape de fluoration peut être comprise entre 10 et 50 heures.
- Eventuellement une étape de finition sous courant d'acide fluorhydrique pur ou dilué par de l'azote à une température pouvant aller jusqu'à 450°C. La durée de l'étape de finition peut être comprise entre 2 et 15 heures.

Pendant cette opération, les précurseurs catalytiques (par exemple halogénures de nickel et de chrome, chromate ou bichromate de nickel, oxyde de chrome) sont transformés en fluorures et/ou oxyfluorures correspondants, ce qui entraîne un dégagement d'eau et/ou d'acide chlorhydrique. L'analyse chimique des éléments (chrome, nickel, fluor, aluminium, oxygène), après cette activation, permet de vérifier la composition minérale du catalyseur.

Un tel catalyseur est décrit dans EP-A-486333, en particulier page 3, lignes 11-48, exemples 1A, 2A et 4A, passages auxquels il y est renvoyé.

Les étapes de déhydrofluoration sont mises en oeuvre à des températures qui peuvent être comprises entre 150°C et 600°C, de préférence entre 300 et 500°C et avantageusement entre 300 et 450°C, notamment entre 300 et 400°C.

Le temps de contact (rapport entre le volume de catalyseur et le flux total de la charge) est en général compris entre 0,1 et 100 secondes, de préférence entre 1 et 50 secondes et avantageusement entre 2 et 20 secondes dans le cas de la réaction conduisant au 1234yf et entre 5 et 40 secondes dans le cas de la réaction conduisant au 1225ye.

Un gaz diluant (azote, hélium, argon) peut être utilisé dans la réaction. La pression dans les différentes réactions peut être atmosphérique, ou inférieure ou supérieure à cette pression atmosphérique. La pression peut varier d'une réaction à l'autre, le cas échéant.

Les réactions sont mises en oeuvre dans un ou plusieurs réacteurs dédiés aux réactions impliquant des halogènes. De tels réacteurs sont connus de l'homme du métier, et peuvent comprendre des revêtements intérieurs à base par exemple de Hastelloy®, Inconel®, Monel® ou de fluoropolymères. Le réacteur peut aussi comprendre des moyens d'échange de chaleur, si besoin.

Dans le cas où l'hydrogène est utilisé en excès dans l'étape d'hydrogénation antérieure à l'étape de déhydrofluoration, de l'hydrogène sera présent lors de la déhydrofluoration. On peut aussi prévoir d'injecter de l'hydrogène dans cette étape (ii), sans pour autant que cet hydrogène provienne de l'étape (i). Le ratio H₂/charge de déhydrofluoration peut varier dans de grandes mesures, notamment entre 0,3 et 30, notamment entre 0,5 et 20, avantageusement entre 1 et 10.

Cette présence d'hydrogène permet d'obtenir une plus grande sélectivité dans le produit recherché; de préférence une sélectivité plus stable dans le temps. De même, la formation de lourds est de préférence réduite. En présence d'hydrogène, la sélectivité est très élevée dans le produit recherché, 1225ye ou 1234yf, sélectivité qui est de préférence stable dans le temps.

En effet, pour les deux réactions de déhydrofluoration, le produit de départ peut être représenté par la formule (I), CF₃-CHF-CHFX, dans lequel X est hydrogène ou fluor. Ainsi, le produit de départ peut correspondre à la formule (Ia) CF₃-CHF-CHF₂ (F236ea) ou à la formule (Ib) CF₃-CHF-CH₂F (F245eb). Dans ces deux cas, l'élimination de HF dans la formule I peut conduire à deux produits, le premier de formule (II) CF₃-CF=CHX et le second de formule (III) CF₃-CH=CFX, selon le fluor qui est éliminé. Il y a donc un problème de sélectivité lors de l'élimination de HF de la molécule du produit de formule (I) . Un tel problème de sélectivité ne se pose pas si le produit de départ ne présente pas de fluor sur le carbone terminal destiné à porter la double liaison, comme par exemple le F245cb, qui ne peut que conduire au 1234yf par élimination de HF.

Lorsque le produit de départ est le produit de formule (Ia) (236ea), le produit recherché correspond à la formule (IIa), à savoir CF₃-CF=CHF (1225ye) tandis que le produit non recherché correspond à la formule (IIIa) à savoir CF₃-CH=CF₂ (1225zc).

Lorsque le produit de départ est le produit de formule (Ib) (245eb), le produit recherché correspond à la formule (IIb), à savoir CF₃-CF=CH₂ (1234yf) tandis que le produit non recherché correspond à la formule (IIIb) à savoir CF₃-CH=CHF (1234ze).

La sélectivité en produit de formule (II), que ce soit (IIa) ou (IIb), est très élevée, supérieure à 90%, de préférence supérieure à 95% et même avantageusement supérieure ou égale à 98%.

La conversion est aussi très élevée. Avantageusement, la conversion est stable dans le temps.

L'alimentation en réactifs se fait en général en continu, ou peut être étagée le cas échéant. Les points pour les éventuel(s) séparation(s) et/ou recyclage(s) sont variables, en tête de procédé ou à des niveaux intermédiaires.

Selon un mode de réalisation, de l'hydrogène est présent lors de l'étape (ii) de déhydrofluoration. Cet hydrogène peut être injecté lors de cette étape, ou peut provenir d'un excès d'hydrogène utilisé dans la première étape qui n'est pas séparé avant l'étape (ii). Selon un mode de réalisation, de l'hydrogène est introduit en excès dans la première réaction, et il est conservé pendant la première étape (ii) de déhydrohalogénation et éventuellement jusqu'à la dernière étape (iv) qui correspond à la seconde déhydrofluoration; cette étape (iv) peut aussi être mise en oeuvre en l'absence d'hydrogène. Par exemple, la quantité totale d'hydrogène utilisée dans le procédé est introduite lors de l'étape (i), le ratio molaire H₂/hexafluoropropylène étant compris entre 2,3 et 30, avantageusement entre 3 et 20. Dans un tel cas, et sur la base de la valeur basse du ratio molaire de 2,3 moles d'hydrogène pour une mole de HFP, il reste 1,3 mole d'hydrogène lors de la première étape de déhydrofluoration (ce qui favorise la sélectivité, avantageusement de façon stable dans le temps) et le ratio H₂/pentafluoropropylène est alors de 1,3 (en admettant que la conversion et la sélectivité de la première étape de déhydrofluoration est quantitative). Une mole d'hydrogène est consommée lors de la réaction d'hydrogénation vers le 245eb et il reste donc 0,3 mole d'hydrogène pour la seconde étape de déhydrofluoration (ce qui favorise à nouveau la sélectivité). Il est aussi possible que la quantité d'hydrogène soit telle qu'elle soit selon un ratio molaire d'environ 2, de sorte que tout l'hydrogène soit consommé et que la dernière réaction (iv) soit mise en oeuvre en l'absence d'hydrogène.(si l'étape (ii) est quantitative).

Les ratios molaires d'hydrogène sont exprimés sur la base de réactions quantitatives (notamment pour les réactions de déhydrohalogénation et en particulier la réaction (ii); les ratios molaires d'hydrogène sont recalculés en fonction de la conversion et de la sélectivité dans le produit recherché).

L'hydrogène peut aussi être introduit de façon étagée, de l'hydrogène additionnel étant introduit avant la seconde hydrogénation ou avant chaque étape de déhydrofluoration si on souhaite que ces étapes soient mises en oeuvre en présence d'hydrogène. Ainsi, la première étape d'hydrogénation peut se faire avec un ratio molaire H₂/hexafluoropropylène de 1,5, et l'excès d'hydrogène restant (environ 0,5 mole d'hydrogène pour une mole de HFP) est conservé dans l'étape (ii) de la première déhydrofluoration. Avant cette étape ou immédiatement après cette étape, il est possible d'ajouter de l'hydrogène pour que le ratio H₂:1225ye soit au moins égal à 1, avantageusement supérieur à 1 (afin que l'étape (iv) finale de déhydrofluoration soit mise en oeuvre en présence d'hydrogène pour améliorer la sélectivité). On peut aussi ajouter dans le milieu réactionnel de l'hydrogène avant chaque étape, si on le souhaite. Il est possible que l'étape (ii) de déhydrofluoration soit mise en oeuvre en présence d'hydrogène tandis que la dernière étape (iv) ne soit pas mise en oeuvre en présence d'hydrogène.

L'hydrogène qui n'a pas été consommé dans une ou plusieurs étapes est avantageusement séparé et recyclé dans le procédé, avantageusement en tête du procédé.

Les réactions d'hydrogénation sont, de préférence, sensiblement quantitatives. Les réactions de déhydrofluoration ne sont pas nécessairement toujours quantitatives, notamment la réaction (ii) de formation du 1225ye n'est pas nécessairement quantitative et il peut rester du 236ea qui n'a pas réagi.

Ce composé 236ea qui n'a pas réagi peut être séparé, soit après l'étape (ii) soit après l'étape (iii) (mais avant l'étape (iv)). Avantageusement la séparation a lieu après l'étape (iii), les températures d'ébullition du 236ea et du 245eb étant de 6°C et 22,7°C respectivement, donc avec un écart de plus de 15°C. La séparation peut intervenir à ces deux moments car la réaction d'hydrogénation (iii) n'affecte sensiblement pas le 236ea. Ce 236ea séparé peut être recyclé dans le procédé. Il peut être recyclé à l'étape (ii) au cours de laquelle il réagit. Il peut aussi être recyclé en tête du procédé, à l'étape (i) et servir comme diluant au cours de cette étape. L'action diluante du 236ea permet de régler l'exothermicité de la première réaction d'hydrogénation.

Ce composé 236ea qui n'a pas réagi peut aussi ne pas être séparé et rester dans le procédé, notamment jusqu'à l'étape (iv). Au cours de cette étape (iv) de déhydrofluoration, il se formera alors du 1,2,3,3,3-pentafluoropropène-1 (1225ye) supplémentaire à partir du 236ea n'ayant pas réagi. On peut ensuite séparer les deux composés 1225ye et 1234yf et recycler le 1225ye. Les températures d'ébullition des deux fluoroléfines sont certes proches, mais il est possible de procéder à une séparation de ces deux composés. Le recyclage peut être effectué à l'étape (ii) et/ou à l'étape (iii). Ce 1225ye sera transformé de façon quantitative lors de la réaction d'hydrogénation de l'étape (iii) qui est sensiblement quantitative.

Il est donc possible de gérer les flux des produits dans le procédé selon l'invention en fonction des besoins ou non en séparation. Le HF qui est formé peut être séparé après chaque réaction de déhydrofluoration, soit entre ces deux réactions, soit seulement en fin de procédé. Le HF peut être séparé par lavage ou par distillation. Les azéotropes éventuellement formés avec HF peuvent aussi être séparés après l'étape au cours de laquelle ils sont formés ou après une étape ultérieure ou à la fin du procédé. Ces étapes de séparation sont donc placées dans le procédé en fonction des différents besoins. Il est possible aussi de prévoir des recyclages de certains composés séparés seulement (par ex. le 236ea n'ayant pas réagi par exemple), tandis que les autres composants séparés sont envoyés vers d'autres procédés.

Avantageusement, le 1,2,3,3,3-pentafluoropropène-1 (1225ye) n'est pas séparé, ce qui évite de manipuler ce produit qui est toxique. Il est possible d'envoyer le flux de l'étape (ii) directement à l'étape suivante.

Par exemple, on peut avoir un procédé dans lequel les étapes (i), (ii) et (iii) sont mises en oeuvre dans le même réacteur, sur des lits de catalyseurs différents. Avantageusement, dans ce cas, à l'issue de l'étape (iii) on procède à une séparation et éventuelle élimination d'HF, on récupère un flux de 1,1,1,2,3,3-hexafluoropropane qui est recyclé à l'entrée du procédé et on récupère un flux de 1,1,1,2,3-pentafluoropropane et éventuellement (mais de préférence) d'hydrogène qui sont envoyés vers l'étape (iv). Le 1,2,3,3,3-pentafluoropropène-1 (1225ye) n'est pas séparé car il est transformé dans le réacteur en 245eb, ce qui évite de manipuler ce produit 1225ye qui est toxique. On peut aussi prévoir trois réacteurs directement en série, le flux sortant d'un réacteur étant envoyé directement dans le réacteur suivant sans séparation.

Dans le cas visé ci-dessus d'un seul réacteur, le réacteur peut contenir trois espèces catalytiques distinctes, avec des fonctions différentes. L'hydrogénation de l'HFP est effectuée sur un premier lit catalytique (conversion totale et sélectivité quasi 100%). Le 236ea et l'excès d'hydrogène passent alors sur un second lit catalytique, à une température appropriée (le chauffage peut être électrique, par exemple). Les produits de réaction sont alors le 1225ye, du HF, de l'hydrogène en excès et éventuellement du 236ea n'ayant pas réagi. Ceux-ci sont alors envoyés sur un troisième lit catalytique dans lequel se produit une hydrogénation (conversion totale et sélectivité quasi 100%).

Dans les cas ci-dessus d'un seul réacteur ou de trois réacteurs immédiatement en série, on obtient alors un flux de sortie contenant du 245eb, éventuellement de l'hydrogène en excès, HF avec éventuellement des azéotropes, et éventuellement le 236ea n'ayant pas réagi présent avant l'étape d'hydrogénation. On sépare l'hydrogène qui est recyclé en tête du réacteur (ou à un autre niveau dans le procédé) et le 236ea du 245eb. Le 236ea peut être recyclé aussi à l'entrée du réacteur. HF et éventuellement les azéotropes sont aussi séparés (éventuellement en partie par lavage).

Il est aussi possible dans le procédé de prévoir que les étapes d'hydrogénation (i) et (iii) sont mises en oeuvre dans le même réacteur, de préférence avec le même catalyseur, et/ou que les étapes de déhydrofluoration (ii) et (iv) sont mises en oeuvre dans le même réacteur, de préférence avec le même catalyseur. WO 2007/117391 décrit la co-déhydrofluoration de 236ea et de 245eb pour produire un mélange de 1225ze et de 1234yf. Ces deux composés ne sont pas séparés à l'issu de ce procédé.

La co-hydrogénation est mise en oeuvre dans un premier réacteur, dont le flux de sortie contient le 236ea et le 245eb. Le flux de sortie peut être séparé et le 236ea est envoyé dans un premier réacteur de déhydrofluoration, tandis que le 245eb est envoyé dans un second réacteur de déhydrofluoration. Le flux de sortie du premier réacteur de déhydrofluoration contient majoritairement du 1225ye et éventuellement du 236ea n'ayant pas réagi. On peut renvoyer le flux de sortie du premier réacteur de déhydrofluoration vers le réacteur d'hydrogénation, produisant ainsi le composé 245eb à partir de ce 1225ye. Le 236ea éventuellement séparé peut être recyclé en tête de ce réacteur de déhydrofluoration.

On peut aussi procéder en envoyant le flux de sortie du réacteur d'hydrogénation (contenant 245eb et 236ea) ou la combinaison des deux flux des deux réacteurs de d'hydrogénation directement vers un réacteur unique de déhydrofluoration. Le flux de ce réacteur de déhydrofluoration contient du 1234yf, mais aussi du 236ea n'ayant pas réagi et du 1225ye résultant de la déhydrofluoration de 236ea. Ce flux est séparé et on récupère le 1234yf, ainsi que le 1225ye et le 236ea. Comme indiqué ci-dessus on peut séparer les deux fluoroléfines. Il est possible que le 236ea soit recyclé en tête du réacteur de déhydrofluoration tandis que le flux de 1225ye est recyclé en tête du réacteur d'hydrogénation. Il est aussi possible de recycler ces deux composés en tête du ou des réacteur(s) d'hydrogénation.

On rappellera que:
- le taux de conversion est le % du produit de départ ayant réagi (nombre mole de produit départ ayant réagi/nombre mole produit départ introduit);
- la sélectivité en produit recherché est le ratio nombre de mole de produit recherché formé/nombre mole produit départ ayant réagi;
- le rendement en produit recherché est le ratio nombre de mole de produit recherché formé/nombre mole produit départ introduit, le rendement en produit recherché pouvant encore être défini comme le produit de la conversion et de la sélectivité.
- le temps de contact est l'inverse de la vitesse spatiale VVH (ou GHSV en langue anglaise)
- la vitesse spatiale est le rapport entre le débit volumique du flux gazeux total sur le volume du lit catalytique, dans les conditions normales de température et de pression.
- La productivité est exprimée en masse de produit recherché obtenu par unité de temps et par unité de catalyseur (masse ou volume); cette productivité est liée au temps de contact.

### EXEMPLES

Les exemples suivants illustrent l'invention sans la limiter. Exemple 1. Hydrogénation de HFP en 236ea.

On utilise un réacteur contenant 10 g de catalyseur sous forme d'un lit fixe de 16 cm³,. Le catalyseur est un catalyseur du type pastille de Pd2%/C. La pression est de 1 bar.

On obtient alors les résultats suivants (RM signifie ratio molaire).

| Temp. | Temps contact | RMH₂/HFP | Conversion | Sélectivité 236ea | Impuretés organ. | Productivité 236ea h/m³ cata |
|---|---|---|---|---|---|---|
| 120°C | 3,8 sec | 5 | 100% | 99% | 0,8 | 1050 |

### Exemple 2. Déhydrofluoration de 236ea en 1225ye.

### Préparation du catalyseur de déhydrofluoration.

Le catalyseur utilisé est un catalyseur Ni-Cr/AlF₃ préparé comme suit.

Dans un évaporateur rotatif, on place 343 g d'un support obtenu dans une étape précédente par fluoration d'alumine GRACE HSA en lit fixe vers 280°C à l'aide d'air et d'acide fluorhydrique (concentration volumique de 5 à 10% de cet acide dans l'air). L'alumine GRACE HSA de départ présente les caractéristiques physicochimiques suivantes:
- Forme : billes de 0,5-2 mm de diamètre
- surface BET : 220 m²/g
- volume poreux : 1,3 cm³/g

On prépare par ailleurs deux solutions aqueuses séparées:
(a) solution chromique additionnée de chlorure de nickel contenant:

| | |
|---|---|
| - anhydride chromique | 55 g |
| - chlorure de nickel hexahydraté | 130 g |
| - eau | 63 g |

(b) Solution méthanolique contenant:

| | |
|---|---|
| - méthanol | 81 g |
| - eau | 8 g |

Ces deux solutions sont introduites simultanément à une température de 40°C sous pression atmosphérique et en 2 heures environ, sur le support en agitation. Après une étape de maturation sous azote, le catalyseur est séché sous azote, puis sous vide à 65°C puis vers 90°C pendant 6 heures.

On charge 500 g de solide imprégné dans un réacteur tubulaire en inconel. Le catalyseur est tout d'abord séché sous balayage d'azote à basse température puis jusqu'à 320°C, à pression atmosphérique. Il est ensuite fluoré en présence d'un mélange acide fluorhydrique / azote (concentration volumique de 5 à 10% de cet acide dans l'azote) à 320°C puis jusqu'à 390°C. L'alimentation d'HF est alors coupée. Le balayage à l'azote est maintenu pendant 15 minutes à 390°C puis le catalyseur est refroidi jusqu'à 60°C sous balayage d'azote.

Les caractéristiques du catalyseur après activation sont les suivantes:
- surface BET : 40 m²/g
- volume poreux : 0,4 cm³/g
- composition chimique pondérale:
   ▪ Al : 25 %
   ▪ F : 58 %
   ▪ Cr : 5,3 %
   ▪ Ni : 6,4 %

On utilise un réacteur contenant 20 g de catalyseur sous forme d'un lit fixe de 23 cm³. La pression est de 1 bar.

On obtient alors les résultats suivants (RM signifie ratio molaire).

| Temp. | Temps contact | RM H₂/236ea | Conversion | Sélectivité 1225ye | Ratio Z/E | Productivité 1225ye h/m³ cata |
|---|---|---|---|---|---|---|
| 375°C | 6,6 sec | 4, 05 | 49,5% | 98% | 7,3 | 310 |

### Exemple 3. Hydrogénation de 1225ye en 245eb.

On utilise un réacteur contenant 10g de catalyseur (identique à celui utilisé à l'exemple 1) sous forme d'un lit fixe de 16 cm³. La pression est de 1 bar.

On obtient alors les résultats suivants (RM signifie ratio molaire).

| Temp. | Temps contact | RM H₂/1225 | Conversion | Sélectivité 236ea | Impuretés organ. | Productivité 245eb h/m³ cata |
|---|---|---|---|---|---|---|
| 20/60°C | 4,6 sec | 8,5 | 100% | 99,5% | 0,5 | 450 |

### Exemple 4. Déhydrofluoration de 245eb en 1234yf.

On utilise un réacteur contenant 10 g de catalyseur (identique à celui de l'exemple 2) sous forme d'un lit fixe de 12 cm³. La pression est de 1 bar.

On obtient alors les résultats suivants (RM signifie ratio molaire).

| Temp. | Temps contact | RM H₂/245eb | Conversion | Sélectivité 1234yf | Sélectivité 1234ze | Productivité 1234yf h/m³ cata |
|---|---|---|---|---|---|---|
| 373°C | 8,9 sec | 3 | 87% | 90% | 9% | 436 |

## Revendications

1. Procédé continu en phase gaz de préparation de 2,3,3,3-tétrafluoro-1-propène comprenant les étapes suivantes:
(i) hydrogénation de hexafluoropropylène en 1,1,1,2,3,3-hexafluoropropane;
(ii) déhydrofluoration du 1,1,1,2,3,3-hexafluoropropane obtenu à l'étape précédente en 1,2,3,3,3-pentafluoropropène-1;
(iii) hydrogénation du 1,2,3,3,3-pentafluoropropène-1 obtenu à l'étape précédente en 1,1,1,2,3-pentafluoropropane;
(iv) déhydrofluoration du 1,1,1,2,3-pentafluoropropane obtenu à l'étape précédente en 2,3,3,3-tétrafluoro-1-propène.

2. Procédé selon la revendication 1, dans lequel l'hydrogène est introduit lors de l'étape (i) et/ou l'étape (iii) selon un ratio molaire surstoechiométrique.

3. Procédé selon la revendication 1 ou 2, dans lequel l'étape (ii) est mise en oeuvre en présence d'hydrogène, de préférence avec un ratio molaire H₂/produit à réagir compris entre 0,3 et 30, notamment entre 0,5 et 20, avantageusement entre 1 et 10, et l'étape (iv) est mise en oeuvre en l'absence d'hydrogène.

4. Procédé selon la revendication 1 ou 2, dans lequel l'étape (ii) est mise en oeuvre en présence d'hydrogène, de préférence avec un ratio molaire H₂/produit à réagir compris entre 0,3 et 30, notamment entre 0,5 et 20, avantageusement entre 1 et 10, et l'étape (iv) est mise en oeuvre en présence d'hydrogène, de préférence avec un ratio molaire H₂/produit à réagir compris entre 0,3 et 30, notamment entre 0,5 et 20, avantageusement entre 1 et 10.

5. Procédé selon l'une des revendications 1 à 4, dans lequel la quantité totale d'hydrogène est introduite lors de l'étape (i).

6. Procédé selon la revendication 5, dans lequel le ratio molaire H₂/hexafluoropropylène est compris entre 2,3 et 30, avantageusement entre 3 et 20.

7. Procédé selon la revendication 5, dans lequel le ratio molaire H₂/hexafluoropropylène est d'environ 2.

8. Procédé selon l'une des revendications 1 à 7, dans lequel le 1,1,1,2,3,3-hexafluoropropane n'ayant pas réagi au cours de l'étape (ii) est séparé après l'étape (ii) ou après l'étape (iii), mais avant l'étape (iv), et est éventuellement recyclé à l'étape (ii) et/ou à l'étape (i).

9. Procédé selon l'une des revendications 1 à 7, dans lequel le 1,1,1,2,3,3-hexafluoropropane n'ayant pas réagi au cours de l'étape (ii) n'est pas séparé avant les étapes (iii) et (iv), du 1,2,3,3,3-pentafluoropropène-1 supplémentaire est obtenu au cours de l'étape (iv) à partir du 1,1,1,2,3,3-hexafluoropropane n'ayant pas réagi et ce 1,2,3,3,3-pentafluoropropène-1 supplémentaire est ensuite séparé et éventuellement recyclé à l'étape (iii) et/ou éventuellement l'étape (ii).

10. Procédé selon l'une des revendications 1 à 9, dans lequel les étapes d'hydrogénation (i) et (iii) sont mises en oeuvre dans le même réacteur, de préférence avec le même catalyseur, une étape de séparation étant éventuellement présente.

11. Procédé selon l'une des revendications 1 à 10, dans lequel les étapes de déhydrofluoration (ii) et (iv) sont mises en oeuvre dans le même réacteur, de préférence avec le même catalyseur, et dans lequel le procédé comprend en outre une étape de séparation séparant les produits issus dudit réacteur, notamment en une fraction contenant le 2,3,3, 3-tétrafluoro-1-propène.

12. Procédé selon l'une des revendications 1 à 9, dans lequel le flux de l'étape (ii) contenant le 1,2,3,3,3-pentafluoropropène-1 est envoyé directement sans séparation vers l'étape (iii) au cours de laquelle se produit d'hydrogénation.

13. Procédé selon l'une des revendications 1 à 9, dans lequel les étapes (i), (ii) et (iii) sont mises en oeuvre dans le même réacteur, sur des lits de catalyseurs différents.

14. Procédé selon l'une des revendications 1 à 9, dans lequel les étapes (i), (ii) et (iii) sont mises en oeuvre dans trois réacteurs immédiatement en série sans séparation intermédiaire.

15. Procédé selon la revendication 13 ou 14, dans lequel à l'issue de l'étape (iii) on procède à une séparation, on récupère un flux de 1,1,1,2,3,3-hexafluoropropane et éventuellement de HF qui est recyclé à l'entrée du procédé et on récupère un flux de 1,1,1,2,3-pentafluoropropane et éventuellement d'hydrogène qui sont envoyés vers l'étape (iv).
